# FASCICULE DE BREVET EUROPEEN

(11) **EP 1 586 438 B1**
(45) Date de publication et mention de la délivrance du brevet: **18.04.2007**
(21) Numéro de dépôt: 05102851.2
(22) Date de dépôt: 12.04.2005
(51) Int. Cl.: B29D 30/06, B60C 11/00

(54) **Moule et bande de roulement confectionnée avec ce moule**
Reifenform und mit dieser Reifenform hergestellter Reifenlaufstreifen
Tire mould and tread made by this mould

(30) Priorité: 13.04.2004 FR 0403907
(43) Date de publication de la demande: 19.10.2005
(73) Titulaire: Société de Technologie Michelin, 63000 Clermont-Ferrand Cedex 09 (FR); MICHELIN Recherche et Technique S.A., 1763 Granges-Paccot (CH)
(72) Inventeur: De Benedittis, Eric, 63360 Saint Beauzire (FR); Menard, Gilbert, 63530 Volvic (FR)
(74) Mandataire: Diernaz, Christian

(56) Documents cités:
- EP-A- 1 232 852
- GB-A- 264 073
- GB-A- 266 885
- US-A- 1 604 450
- US-A1- 2003 201 048

## Description

### Domaine de l'invention

La présente invention concerne les moules et les bandes de roulement pour pneumatiques. En particulier, elle concerne les moules permettant de mouler des bandes de roulement comportant des cavités dont au moins une partie est située sous la surface de roulement et qui débouchent sur au moins une face latérale de cette même bande de roulement.

### Définitions

Dans le présent document, les termes « axial » et « axialement » se réfèrent à une direction sensiblement parallèle à l'axe de rotation d'un pneumatique. Lorsque ces termes sont appliqués à une bande de roulement, ils se réfèrent à la direction qui est parallèle à l'axe de rotation du pneumatique lorsque la bande de roulement est fixée sur le pneumatique. Autrement dit, la direction axiale est la direction perpendiculaire à la fois à l'épaisseur de la bande de roulement et à la circonférence du pneumatique.

Les termes « radial » et « radialement » se réfèrent à une direction parallèle à un vecteur perpendiculaire à la direction axiale et qui coupe l'axe de rotation d'un pneumatique. Lorsque ces termes sont appliqués à une bande de roulement, ils se réfèrent aux directions parallèles à un vecteur perpendiculaire à l'axe de rotation du pneumatique et comprenant un point de l'axe de rotation du pneumatique, lorsque la bande de roulement est incorporée sur le pneumatique. Autrement dit, une direction radiale est une direction parallèle à l'épaisseur de la bande de roulement.

Par « surface de roulement » d'une bande de roulement, on entend la surface formée par les points de la bande de roulement qui entrent en contact avec le sol lorsque le pneumatique roule.

Par « face latérale » d'une bande de roulement, on entend toute partie de la surface de la bande de roulement qui s'étend des extrémités axiales de la surface de roulement aux flancs du pneumatique. Lorsqu'on considère une bande de roulement avant d'être incorporée sur un pneumatique, une face latérale est constituée par la partie de la surface de la bande de roulement qui relie un des bords axiaux de la surface de roulement à la surface destinée à entrer en contact avec la carcasse du pneumatique.

Par « doigt », on entend tout élément moulant destiné à mouler une cavité dont au moins une partie est située sous la surface d'un moule moulant une surface de roulement et qui débouche sur au moins une face latérale de la bande de roulement, sans limitation de la géométrie.

Par « axe longitudinal » d'un doigt, on entend un axe sensiblement parallèle à la direction de la plus grande dimension du doigt.

Par « mélange » ou « mélange de caoutchouc », on entend une composition de caoutchouc comportant au moins un élastomère et une charge.

Par « flux » de mélange, on entend l'écoulement d'une quantité de mélange lors du moulage.

Par « dissymétrie des flux » créée lors du moulage d'une cavité, on entend la dissymétrie dans le champ des vitesses d'écoulement entre les parties du mélange qui contournent le doigt moulant la cavité de part et d'autre de la surface perpendiculaire à la surface du moule moulant la surface de roulement et comprenant les points du doigt qui se trouvent au plus loin de la surface du moule moulant la surface de roulement, comme décrit plus bas.

Le terme « pneumatique » désigne ici tous types de bandages élastiques, gonflés ou non, soumis en service à une pression interne ou non.

### Arrière-plan

La présence de cavités situés sous la surface de roulement d'un pneumatique et débouchant sur au moins une face latérale de la bande de roulement confère des propriétés intéressantes au pneumatique, notamment lorsqu'il comporte une bande de roulement dont l'épaisseur maximale est supérieure à 15 mm. Les cavités contribuent au refroidissement des épaules du pneumatique (effet de ventilation) et améliorent par conséquent son endurance. De plus, elles rendent la sculpture de la bande de roulement évolutive, car elles apparaissent en surface de la bande de roulement au fur et à mesure de son usure, favorisant ainsi l'adhérence sur sol humide, sur neige et sur glace.

Des moules permettant de mouler des bandes de roulement pourvues de ce type de cavités sont connus depuis longtemps. Deux approches peuvent être distinguées : soit on fait pénétrer des doigts mobiles dans le mélange du pneumatique cru, soit on oblige, lors du moulage, le mélange qui formera la bande de roulement à fluer et à envelopper ainsi les doigts.

Le brevet US 1 604 450 est un exemple de la première approche : il décrit l'utilisation de doigts amovibles qui pénètrent axialement dans une face latérale de la bande de roulement après la mise en place du pneumatique cru dans le moule et avant la cuisson du pneumatique. Les doigts doivent pénétrer sans se déformer mais aussi sans déplacer ni déformer le pneumatique cru, ce qui impose des contraintes en terme de géométrie et d'épaisseur (résistance mécanique) des doigts.

Les moules correspondant à la deuxième approche permettent le moulage des cavités sans mouvement des doigts pendant la phase de moulage. Le brevet US 6 408 910 décrit un moule dont les doigts sont solidaires des coquilles prévues pour mouler les flancs d'un pneumatique. Lors du moulage, la pression interne au pneumatique cru - et donc déformable plastiquement - exercée par la membrane provoque l'augmentation du diamètre extérieur du pneumatique cru. Cette étape appelée « conformation » oblige la matière constituant la bande de roulement à venir remplir tous les espaces du moule pour créer la bande de roulement finale comportant les cavités. Pour démouler, les coquilles sont écartées axialement, ce qui permet l'extraction des doigts de la bande de roulement. Ce procédé présente un inconvénient : il est réservé aux fabrications dans lesquelles la conformation est relativement importante, car elle doit correspondre au minimum à l'épaisseur de la sculpture prévue, y compris les cavités sous la surface de roulement.

Or la tendance de l'industrie est de chercher à limiter au maximum l'importance de la conformation. C'est la raison pour laquelle la demande de brevet EP 1 232 852 présente un autre type de moule, correspondant à la première approche. Le moule proposé comprend une série de secteurs de couronne, destinés à mouler des cavités ou rainures radiales, et une série de secteurs d'épaule comportant des doigts destinés à mouler les cavités axiales, les deux séries de secteurs étant mobiles radialement. La disposition des doigts sur des secteurs mobiles rend possible l'agrandissement du diamètre du cercle sur lequel sont disposés les doigts avant la mise en place du bandage cru, ce qui permet de réduire la conformabilité du bandage. Il est proposé que les doigts prennent appui, pendant la phase de pénétration radiale dans le mélange, sur les secteurs de couronne afin de résister à la pression exercée par la matière de moulage sur les doigts. Ainsi il devient possible d'utiliser des doigts relativement longs et fins, et donc susceptibles de fléchir sous la pression exercée par le mélange cru.

Les deux approches ont montré leur efficacité en usage ; toutefois, les bandes de roulement ainsi fabriquées peuvent avoir des propriétés indésirables. En effet, quelle que soit l'approche choisie : pénétration radiale du doigt ou fluage radial du mélange, la formation de la bande de roulement finale implique un écoulement du mélange de part et d'autre du doigt et la formation d'un plan de jonction (aussi appelé « soudure ») s'étendant entre la surface du doigt et la surface du moule moulant la bande de roulement du pneumatique. L'utilisation de moules correspondant à l'état de l'art induit un plan de joint perpendiculaire à cette surface, qui peut se révéler fragile, notamment à cause de sa sensibilité aux pollutions lors du moulage.

### Résumé de l'invention

Un objectif de la présente invention est de piloter le positionnement de la zone de jonction et son orientation par rapport à la surface du pneumatique. Il est proposé d'atteindre cet objectif à l'aide d'un moule pour le moulage d'une bande de roulement en mélange de caoutchouc, cette bande ayant une surface de roulement limitée axialement par des faces latérales, ce moule comprenant au moins une partie pour mouler la surface de roulement, au moins deux parties latérales pour mouler les faces latérales et au moins un doigt pour mouler une cavité à l'intérieur de la bande de roulement, ce doigt, vu dans un plan de coupe perpendiculaire à la surface moulant la surface de roulement et à la direction axiale, ayant une surface dont le contour est formé d'une première portion et d'une seconde portion de contour, ces portions de contour s'étendant entre le point du contour situé au plus près et le point du contour situé au plus loin de la surface moulant la surface de roulement, ce doigt étant situé au moins en partie sous la surface moulant la surface de roulement et connecté à au moins une surface moulant au moins une partie d'une face latérale de la bande de roulement, le doigt créant, pendant le moulage, deux flux de mélange de caoutchouc enveloppant le doigt et se rejoignant pour former une surface de jonction, ce moule étant caractérisé en ce qu'au moins un doigt comporte des moyens pour rendre dissymétriques les flux de mélange de façon à ce que la surface de jonction, vue dans ledit plan, soit telle que la longueur de la trace de cette surface de jonction sur ledit plan, mesurée entre la surface du doigt et la surface moulant la surface de roulement soit supérieure à la distance minimale entre la surface du doigt et ladite surface moulant la surface de roulement.

Dans le cas où plusieurs points du contour sont à même distance maximale par rapport à la surface du moule moulant la surface de roulement, on retient les deux points les plus éloignés dans une direction perpendiculaire à la direction radiale et à la direction axiale. Les deux portions de contour s'étendent entre chacun de ces deux points et le point du contour situé au plus près de la surface moulant la surface de roulement. On procède de manière analogue si plusieurs points du contour possèdent la même distance minimale par rapport à la surface du moule moulant la surface de roulement. A titre d'exemple, pour un doigt dont la surface, dans un plan de coupe perpendiculaire à la surface du moule moulant la surface de roulement et à la direction axiale, est un rectangle dont les longueurs sont parallèles à la surface du moule moulant la surface de roulement et dont les largeurs sont perpendiculaires à cette surface du moule, les deux portions de contour coïncident avec les largeurs du rectangle.

Le moule de l'invention permet d'obtenir l'agrandissement de la surface de jonction qui s'étend entre la surface de la cavité et la surface du moule moulant la surface de roulement, quel que soit le degré de conformation ; les moyens proposés peuvent être mis en oeuvre dans des moules avec ou sans conformation.

Plusieurs moyens peuvent être utilisés pour obtenir des flux dissymétriques. On peut notamment choisir une géométrie de doigt susceptible de rendre dissymétriques les flux de mélange lors de la pénétration du doigt ou, dans le cas de la conformation, lors du fluage du mélange autour du doigt.

Selon un mode de réalisation préférentiel, les flux sont rendus dissymétriques par l'utilisation de doigts dont les longueurs desdites première et seconde portions de contour sont différentes. De préférence, la différence des longueurs des première et seconde portions de contour est au moins égale à 10 % de la longueur de la portion de contour la plus courte.

Selon un autre mode de réalisation préférentiel, on rend dissymétriques les flux de mélange autour d'un doigt en conférant à l'une des portions de contour une rugosité moyenne supérieure à la rugosité moyenne de l'autre portion de contour. A titre d'exemple, on peut augmenter la rugosité de la première portion par sablage et réduire la rugosité de la deuxième portion par polissage. De préférence, la différence entre les rugosités Ra moyennes des première et seconde portions de contour est au moins égale à 2 micron. Bien entendu, il est possible de combiner les effets de géométrie et de rugosité de surface en utilisant des doigts dont lesdites première et seconde portions ont à la fois des longueurs et des rugosités différentes.

Selon un troisième mode de réalisation préférentiel, les flux sont rendus dissymétriques par un revêtement chimique appliqué à au moins une partie d'au moins une desdites portions de contour de façon à créer une différence de vitesse de glissement du mélange de caoutchouc sur lesdites portions. A titre d'exemple, on peut revêtir la première portion de contour d'Araldite® et réaliser la deuxième portion en acier. On combinera avantageusement les effets de revêtement et de géométrie et/ou de rugosité en utilisant des doigts dont lesdites première et seconde portions ont à la fois des revêtements et des longueurs et/ou des rugosités différents.

Selon un quatrième mode de réalisation préférentiel, on rend dissymétriques les flux de mélange par un effet de température, en créant une différence de température moyenne entre les surfaces correspondant aux première et seconde portions de contour de chaque doigt. De préférence, l'écart de température est au moins égal à 20° C. Rien ne s'oppose à la combinaison des effets de la température et de la géométrie et/ou la rugosité et/ou le revêtement du doigt pour rendre dissymétriques les flux de mélange autour de chaque doigt de façon optimale.

Selon un cinquième mode de réalisation préférentiel, au moins un doigt pour le moulage d'une cavité dispose de moyens additionnels pour le mettre en rotation sensiblement autour de son axe longitudinal. Cette mise en rotation peut être effectuée pendant de la pénétration du doigt dans le mélange (si les doigts sont mobiles), pendant la phase de conformation (s'il y en a) et/ou lorsque le doigt est entouré de mélange, avant la cuisson de la bande de roulement.

Tous les modes de réalisation décrits plus haut sont applicables dans des moules avec ou sans conformation. Un dernier mode de réalisation préférentiel concerne plus particulièrement les moules sans conformation dont les doigts mobiles pénètrent dans le mélange formant la bande de roulement après l'insertion de l'ébauche du pneumatique ou de la bande de roulement dans le moule. Selon ce mode de réalisation préférentiel, au moins un doigt, de préférence de géométrie cylindrique, est monté axialement et radialement mobile par rapport au moule pour pouvoir effectuer un mouvement de translation dans un plan formant un angle inférieur à 90° avec une direction radiale. Les flux de mélange contournant le doigt pendant sa pénétration dans le mélange se referment après le passage du doigt et forment une surface de jonction qui est inclinée par rapport à un plan perpendiculaire à la surface du moule moulant la surface de roulement. Par conséquent, la longueur moyenne de la trace de la surface de jonction sur ledit plan, mesurée entre la surface du doigt et la surface du moule moulant la surface de roulement, est supérieure à la distance minimale entre cette cavité et la surface du moule moulant la surface de roulement. Il est possible de combiner l'effet de la pénétration selon une direction formant un angle inférieur à 90° avec une direction perpendiculaire à la surface de moulage avec les effets de la température et/ou la géométrie et/ou la rugosité et/ou le revêtement et/ou la rotation du doigt sensiblement autour de son axe longitudinal pour obtenir l'agrandissement de la surface de jonction qui s'étend entre la surface du doigt et la surface du moule moulant la surface de roulement.

L'invention concerne également une bande de roulement en mélange de caoutchouc, cette bande ayant une surface de roulement limitée axialement par des faces latérales et comportant au moins une cavité dont au moins une partie est située sous la surface de roulement et qui débouche sur au moins une face latérale de cette même bande de roulement, cette bande étant pourvue d'une surface de jonction s'étendant entre la surface d'au moins une cavité et la surface de roulement, cette bande étant caractérisée en ce que la longueur de la trace de la surface de jonction, dans un plan de coupe perpendiculaire à la surface de roulement et à la direction axiale, mesurée entre la cavité et la surface de roulement est supérieure à la distance minimale entre la surface de la cavité et ladite surface de roulement. Préférentiellement, la différence entre la longueur de ladite trace et la distance minimale entre la surface de la cavité et la surface de roulement est supérieure à 20% de ladite distance minimale.

L'invention sera mieux comprise grâce à la description des dessins selon lesquels :

la figure 1 représente schématiquement en coupe axiale une partie d'un moule en configuration fermée ainsi que la partie correspondante d'un pneumatique moulé dans ce moule ;

la figure 2 représente schématiquement une coupe radiale AA' du moule de la figure 1, équipé d'un doigt selon l'art antérieur ;

la figure 3 représente schématiquement une coupe radiale AA' du moule de la figure 1, équipé d'un doigt selon l'invention et illustrant la notion de « portion de contour » ;

les figures 4 à 7 représentent schématiquement, dans un plan de coupe radial AA' du moule de la figure 1, la pénétration d'un doigt selon l'invention dans le mélange composant la bande de roulement ;

Les figures 8 à 11 représentent schématiquement des variantes de doigts selon l'invention, vus en coupe.

### Description de modes de réalisation

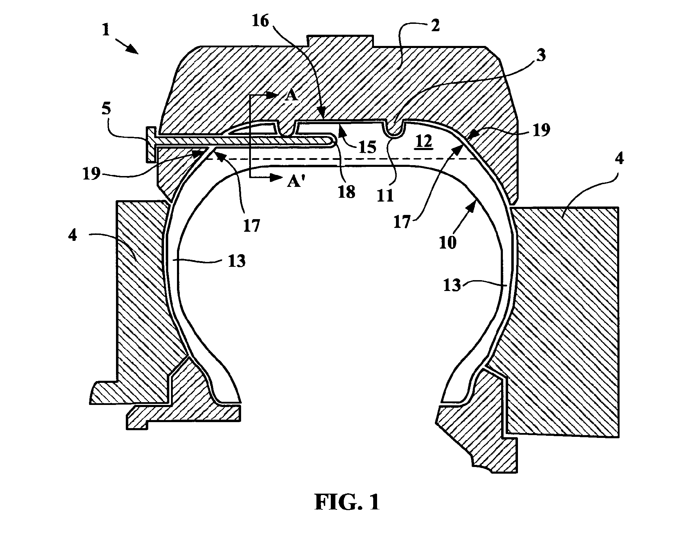

La figure 1 représente un moule 1 selon l'invention dans sa configuration fermée. La représentation est schématique : en particulier, seulement les parties moulantes du moule 1 ont été représentées ; les parties renforçantes ont été omises par souci de clarté. Afin de mieux comprendre les fonctions des éléments décrits, on a représenté également une portion correspondante d'un pneumatique 10 obtenu par ce moule 1. Sur cette vue partielle en coupe selon un plan comprenant l'axe de rotation du pneumatique, on reconnaît un secteur de couronne 2 portant un relief 3 pour mouler une cavité ou une rainure 11 d'une bande de roulement 12 du pneumatique 10. Ce secteur de couronne 2 est mobile radialement par rapport à des coquilles 4 moulant des flancs 13 du pneumatique 10 de manière connue en soi. Le secteur de couronne 2 porte un doigt 5 apte à mouler une cavité 18 s'étendant axialement sous la surface de roulement et débouchant sur une face latérale 17 de la bande de roulement 12. Ce doigt 5 est mobile axialement afin de permettre le démoulage du pneumatique.

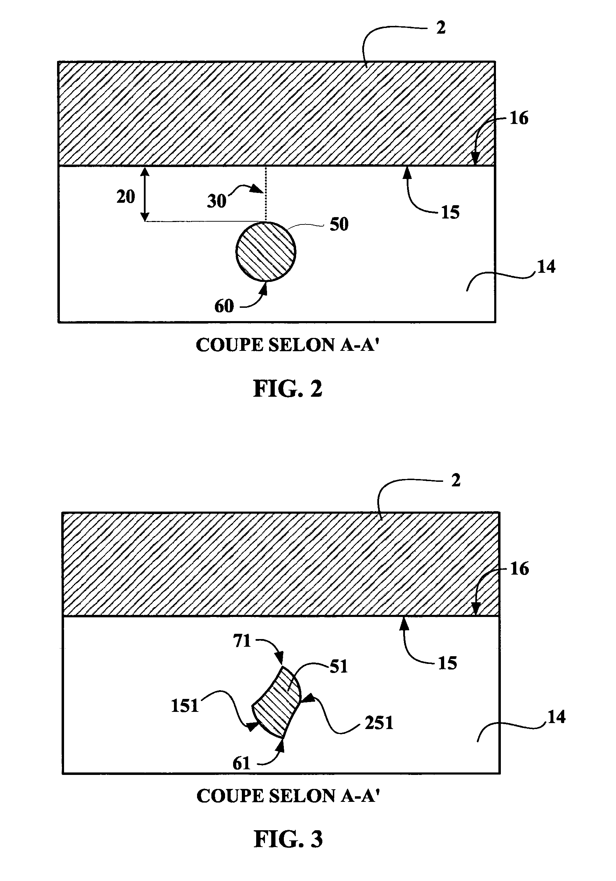

La figure 2 représente schématiquement une coupe radiale AA' du moule 1 de la figure 1, équipé d'un doigt 50 selon l'art antérieur. Ce doigt 50 est entouré de mélange 14 composant la bande de roulement 12. On voit également une partie du secteur de couronne 2. La surface de roulement 15 se situe à l'interface entre la bande de roulement 12 et le secteur de couronne 2.

Le doigt 50 selon l'art antérieur ne rend pas dissymétriques les flux de mélange pendant le moulage de la cavité 18 : les champs de vitesse caractérisant l'écoulement du mélange sont symétriques par rapport à l'axe perpendiculaire à la surface 16 du moule moulant la surface de roulement 15 et passant par le point 60 du doigt, situé au plus loin de cette surface. Par conséquent, la surface de jonction 30 qui se forme entre les parties de mélange ayant contourné le doigt 50 lors du moulage est sensiblement perpendiculaire à la surface 16 du moule moulant la surface de roulement 15. Sa longueur est sensiblement égale à la distance minimale 20 séparant la surface du doigt 50 de la surface 16.

La figure 3 représente schématiquement une coupe radiale AA' du moule 1 de la figure 1, équipé d'un doigt 51 selon l'invention. Ce doigt 51 permet l'illustration de la notion de « portion de contour ». Dans le plan de coupe choisi, on reconnaît facilement le point 61 du contour du doigt 51 le plus éloigné de la surface 16 du moule moulant la surface de roulement 15 ainsi que le point 71 du contour du doigt 51 le plus près de cette même surface, ainsi que les deux portions de contour 151 et 251 qui les relient. Dans le cas présenté, la différence des longueurs des première et seconde portions de contour est égale à 20 % de la longueur de la portion 251.

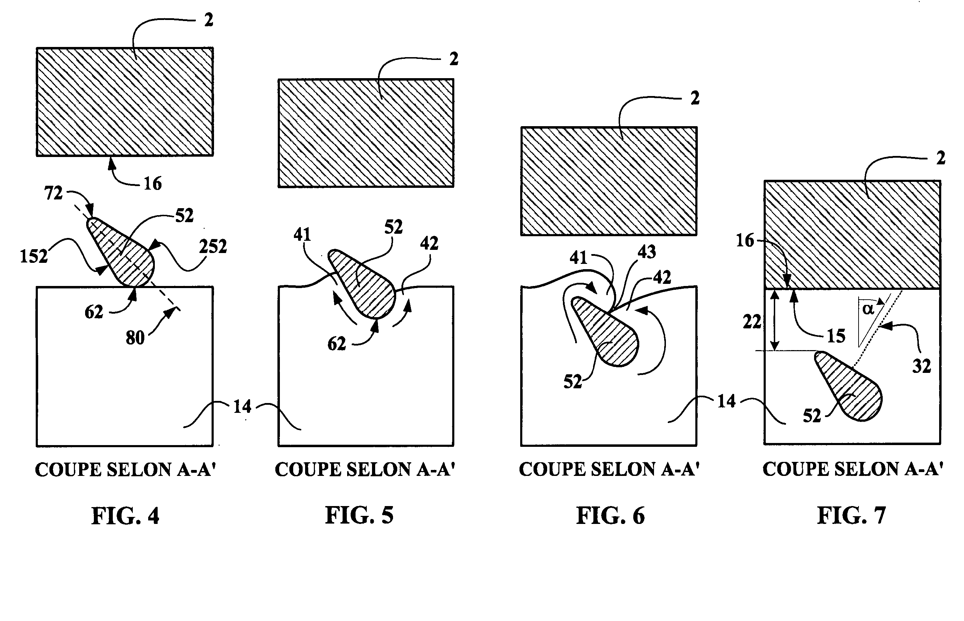

Les figures 4 à 7 représentent schématiquement, dans un plan de coupe AA' du moule 1 de la figure 1, la pénétration d'un doigt 52 selon l'invention dans le mélange 14 composant la bande de roulement 12. Le schéma est valable pour des moules avec conformation (c'est alors le mélange 14 qui est en mouvement et enveloppe le doigt 52) ou sans conformation (dans ce cas, c'est le doigt 52 qui est en mouvement et pénètre dans le mélange 14).

La figure 4 représente une coupe radiale AA' du moule 1 à l'instant où le doigt 52 entre en contact avec le mélange 14 composant la bande de roulement 12. Ce contact se fait au point 62 du doigt 52 le plus éloigne de la surface 16 moulant la surface de roulement 15 (figure 7).

La figure 5 représente la même coupe radiale AA' du moule 1 quelques instants plus tard. Le point 62 du doigt 52 a pénétré dans le mélange 14, créant ainsi deux flux de mélange 41 et 42. On voit que la géométrie du doigt 52 génère des écoulements dissymétriques : la partie 41 du mélange se rapproche de la surface 16 plus vite que la partie 42 du mélange contournant le doigt 52 de l'autre côté. On notera que les flux sont rendus dissymétriques par un doigt qui possède un axe de symétrie 80 (trait tireté, figure 4), mais qui est incliné par rapport à la direction perpendiculaire à la surface 16. Pour l'inclinaison choisie, la différence des longueurs des portions de contour 152 et 252 est égale à 10 % de la longueur de la portion 152. Si le même doigt 52 avait été monté dans le moule de manière à ce que l'axe 80 soit perpendiculaire à la surface 16, les portions 152 et 252 auraient la même longueur et le doigt ne rendrait pas dissymétriques les flux de mélange 41 et 42 le contournant.

La figure 6 représente la même coupe AA' du moule 1 au moment où les flux de mélange 41 et 42 se rejoignent après avoir contourné le doigt 52. Le point de rencontre 43 qui constitue l'amorce de la surface de jointure 32 (figure 7) ne coïncide pas avec le point 72 (figure 4) le plus proche de la surface 16.

La figure 7 représente la même coupe AA' du moule 1 à la fin de la pénétration du doigt 52 dans le mélange 14 composant la bande de roulement 12. Le mélange 14 a enveloppé le doigt 52 et rempli tout l'espace entre le doigt 52 et la surface 16 moulant la surface de roulement 15. Les parties de mélange 41 et 42 ayant contourné le doigt 52 se sont rejoints en formant une surface de jonction 32 s'étendant de la surface du doigt 52 jusqu'à la surface 16 moulant la surface de roulement 15. Le fait que les flux soient dissymétriques génère une inclinaison (α) de la surface de jonction 32. La longueur de la trace de cette surface 32 sur le plan de coupe, mesurée entre la surface du doigt 52 et la surface 16 est supérieure à la distance minimale 22 séparant la surface du doigt 52 et la surface 16.

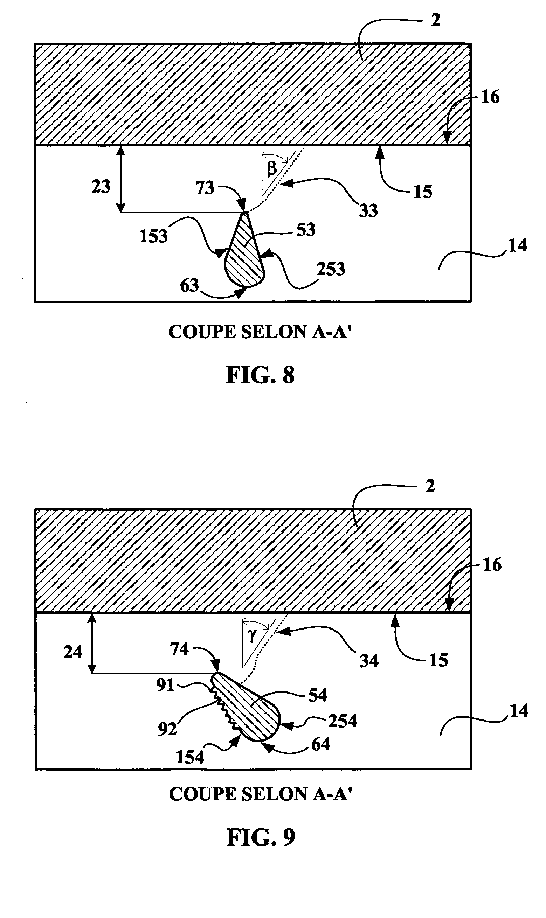

La figure 8 représente schématiquement une coupe radiale AA' du moule 1 de la figure 1, équipé d'un autre doigt 53 selon l'invention. Les flux sont rendus dissymétriques par une géométrie particulière du doigt 53 qui ne possède aucun axe de symétrie dans le plan de coupe choisi. La différence des longueurs des portions de contour 153 et 253, reliant le points le plus éloigné 63 et le point le plus près 73 de la surface 16 moulant la surface de roulement 15, est égale à 10 % de la longueur de la portion 253. Le fait que les flux soient dissymétriques génère une inclinaison (β) de la surface de jonction 33 qui se forme entre les parties mélange ayant contourné le doigt 53 lors du moulage. La longueur de la trace de cette surface 33 sur le plan de coupe, mesurée entre la surface du doigt 53 et la surface 16 moulant la surface de roulement 15 est supérieure à la distance minimale 23 séparant la surface du doigt 53 de la surface 16 moulant la surface de roulement 15.

La figure 9 représente schématiquement une coupe radiale AA' du moule 1 de la figure 1, équipé d'un quatrième doigt 54 selon l'invention. Comme dans le cas précédent, les flux de mélange sont rendus dissymétriques par la géométrie du doigt 54. Ce doigt possède une géométrie proche de celle du doigt 52, à la différence près que l'une des portions de contour est pourvue de nervures 91 et rainures 92 axiales, disposées parallèlement les unes aux autres avec un pas de 3 mm et ayant un profil triangulaire d'une hauteur de 2 mm. La différence des portions de contour 154 et 254 reliant le points le plus éloigné 64 et le point le plus près 74 de la surface 16 moulant la surface de roulement 15 est égale à 10 % de la longueur de la portion 154. Le fait que les flux soient dissymétriques génère une inclinaison moyenne (γ) de la surface de jonction 34 qui se forme entre les parties de mélange ayant contourné le doigt 54 lors du moulage. La longueur de la trace de cette surface 34 sur le plan de coupe, mesurée entre la surface du doigt 54 et la surface 16 moulant la surface de roulement 15 est supérieure à la distance minimale 24 séparant la surface du doigt 54 de la surface 16 moulant la surface de roulement 15.

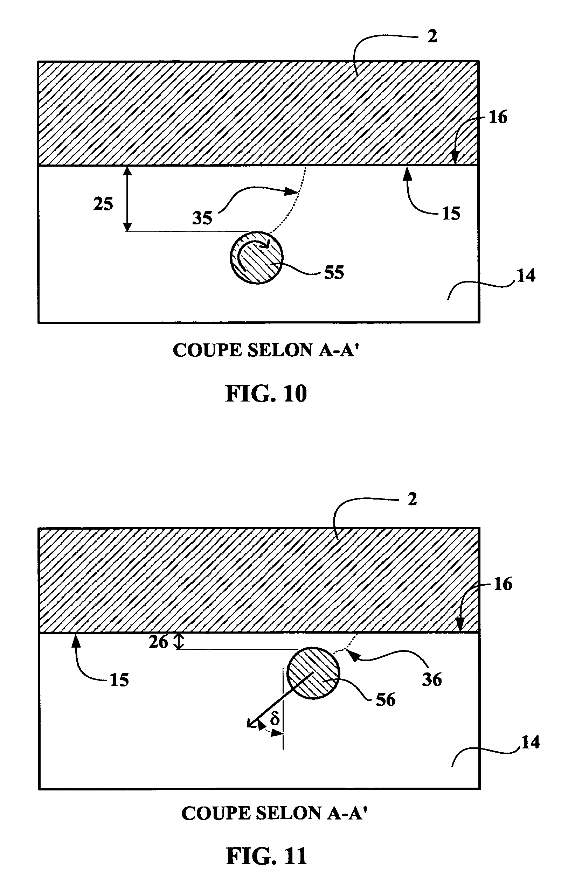

La figure 10 représente schématiquement la coupe radiale AA' du moule 1 de la figure 1, équipé d'un cinquième doigt 55 selon l'invention. Contrairement aux exemples cités plus haut, les flux de mélange sont rendus dissymétriques non pas par la géométrie du doigt 55 mais par la mise en rotation de celui-ci pendant le moulage. Cette rotation rend dissymétrique le champ des vitesses : le mélange qui contourne le doigt est soit accélérée, soit freinée dans son avancement par le mouvement de rotation du doigt 55. Par conséquent, on observe l'inclinaison la surface de jonction 35 qui se forme entre les parties de mélange ayant contourné le doigt 55 lors du moulage. La longueur de la trace de cette surface 35 sur le plan de coupe, mesurée entre la surface du doigt 55 et la surface 16 moulant la surface de roulement 15 est supérieure à la distance minimale 25 séparant la surface du doigt 55 de la surface 16 moulant la surface de roulement 15.

La figure 11 représente schématiquement la coupe radiale AA' du moule 1 de la figure 1, équipé d'un doigt 56 selon l'invention, monté axialement et radialement mobile par rapport au moule 1 selon une direction formant un angle (δ) avec une direction radiale. La direction du mouvement de translation du doigt est indiquée par une flèche. Les flux de mélange contournant de part et d'autre le doigt cylindrique pendant sa pénétration dans le mélange se referment après le passage du doigt et forment une surface de jonction 36 qui est inclinée par rapport à un plan perpendiculaire à la surface 16 moulant la surface de roulement 15. A tout instant la longueur dans le plan de coupe, de la trace de cette surface de jonction 36 est supérieure à la distance minimale 26 séparant la surface du doigt 56 de la surface 16.

On obtient des résultats satisfaisants dès lors que les flux de mélange sont rendus dissymétriques sur la quasi totalité du doigt, à l'exception éventuellement des extrémités du doigt.

Tandis que la figure 1 montre un doigt 5 qui moule une cavité 18 s'étendant d'une face latérale 17 de la bande de roulement 12 du pneumatique 10 jusqu'à mi-largeur de la bande de roulement, on comprend que la cavité 18 peut s'étendre au-delà du milieu de la bande de roulement. Des cavités plus courtes sont également envisageables.

De même, on comprend que le principe de l'invention s'applique indifféremment au cas où l'on désire mouler des cavités sur une face latérale 17 de la bande de roulement seulement ou sur les deux faces latérales. Les cavités sur des flancs opposés peuvent être arrangées de façon symétrique ou non. Les secteurs de couronne 2 peuvent couvrir toute la largeur de la bande de roulement ou seulement une partie de celle-ci.

Le nombre de doigts 5 et leur géométrie précise sont déterminés en fonction du résultat recherché sur la bande de roulement finie. Chaque secteur peut porter plusieurs doigts ou au contraire, certains secteurs peuvent ne pas porter de doigt.

Il est également possible de faire porter les doigts 5 par des secteurs d'épaule mobiles radialement, à la manière décrite dans le document EP 1 232 852.

Des doigts peuvent être disposés axialement ou selon une direction oblique par rapport à l'axe du pneumatique. De même, la section, dans une coupe radiale, d'au moins un doigt peut varier dans la direction de la plus grande dimension du doigt.

Les moules selon l'invention permettent de mouler des bandes de roulement annulaires ou non, de longueur finie ou au contraire de longueur quasi infinie, en mode continu et à plat. On peut de cette manière mouler non seulement des bandes de roulement destinées à la production ou au rechapage de pneumatiques, mais également des chenilles en caoutchouc.

## Revendications

1. - Moule (1) pour le moulage d'une bande de roulement (12) en mélange de caoutchouc, cette bande (12) ayant une surface de roulement (15) limitée axialement par des faces latérales (17), ce moule (1) comprenant au moins une partie (16) pour mouler la surface de roulement (15), au moins deux parties latérales (19) pour mouler les faces latérales (17) et au moins un doigt (5 ; 51 ; 52 ; 53 ; 54 ; 55 ; 56) pour mouler une cavité (18) à l'intérieur de la bande de roulement (12), ce doigt, vu dans un plan de coupe perpendiculaire à la surface (16) moulant la surface de roulement (15) et à la direction axiale, ayant une surface dont le contour est formé d'une première portion (151 ; 152 ; 153 ; 154) et d'une seconde portion (251 ; 252 ; 253 ; 254) de contour, ces portions de contour s'étendant entre le point (71 ; 72 ; 73 ; 74) du contour situé au plus près et le point (61 ; 62 ; 63 ; 64) du contour situé au plus loin de la surface (16) moulant la surface de roulement (15), ce doigt (5 ; 51 ; 52 ; 53 ; 54 ; 55 ; 56) étant situé au moins en partie sous la surface (16) moulant la surface de roulement (15) et connecté à au moins une surface moulant au moins une partie d'une face latérale (17) de la bande de roulement (12), le doigt (5 ; 51 ; 52 ; 53 ; 54 ; 55 ; 56) créant, pendant le moulage, deux flux de mélange de caoutchouc enveloppant le doigt (5 ; 51 ; 52 ; 53 ; 54 ; 55 ; 56) et se rejoignant pour former une surface de jonction (32 ; 33 ; 34 ; 35 ; 36), ce moule étant **caractérisé en ce qu**'au moins un doigt (5 ; 51 ; 52 ; 53 ; 54 ; 55 ; 56) comporte des moyens pour rendre dissymétriques les flux de mélange de façon à ce que la surface de jonction (32 ; 33 ; 34 ; 35 ; 36), vue dans ledit plan, soit telle que la longueur de la trace de cette surface de jonction (32 ; 33 ; 34 ; 35 ; 36) sur ledit plan, mesurée entre la surface du doigt (5 ; 51 ; 52 ; 53 ; 54 ; 55 ; 56) et la surface (16) moulant la surface de roulement (15) soit supérieure à la distance minimale (22 ; 23 ; 24 ; 25 ; 26) entre la surface du doigt (5 ; 51; 52 ; 53 ; 54 ; 55 ; 56) et ladite surface (16) moulant la surface de roulement (15).

2. - Moule selon la revendication 1, **caractérisé en ce que** la longueur de ladite première portion (151 ; 152 ; 153 ; 154) est différente de la longueur de ladite seconde portion (251 ; 252 ; 253; 254).

3. - Moule selon la revendication 2, **caractérisé en ce que** la différence entre la longueur de la première portion (151 ; 152 ; 153 ; 154) et la longueur de la seconde portion (251 ; 252 ; 253 ; 254) de contour est au moins égale à 10 % de la longueur de la portion de contour la plus courte.

4. - Moule selon l'une des revendications 1 à 3, **caractérisé en ce que** l'une des portions de contour est pourvue d'une rugosité moyenne supérieure à la rugosité moyenne de l'autre portion de contour.

5. - Moule selon la revendication 4, **caractérisé en ce que** la différence entre les rugosités Ra moyennes de la première portion (151 ; 152 ; 153 ; 154) et de la seconde portion (251 ; 252 ; 253 ; 254) de contour est au moins égale à 2 micron.

6. - Moule selon l'une des revendications 1 à 5, **caractérisé en ce qu**'au moins une portion de contour est au moins partiellement recouverte d'un revêtement de façon à créer une différence de vitesse de glissement du mélange sur lesdites portions de contour.

7. - Moule selon l'une des revendications 1 à 6, **caractérisé en ce que** des moyens sont en outre prévus pour créer une différence de température moyenne entre la surface de la première portion (151 ; 152 ; 153 ; 154) et la surface de la seconde portion (251 ; 252 ; 253 ; 254) de contour d'au moins un doigt (5 ; 51 ; 52 ; 53 ; 54 ; 55 ; 56).

8. - Moule selon la revendication 7, **caractérisé en ce que** la différence de température est au moins égal à 20° C.

9. - Moule selon la revendication 1, **caractérisé en ce qu**'au moins un doigt (5 ; 51 ; 52 ; 53 ; 54; 55 ; 56) pour le moulage d'une cavité (18) dispose de moyens additionnels pour le mettre en rotation sensiblement autour de son axe longitudinal.

10. - Moule selon l'une des revendications 1 à 9, **caractérisé en ce qu'**au moins un doigt (5 ; 51; 52 ; 53 ; 54 ; 55 ; 56) est monté axialement et radialement mobile par rapport au moule pour pouvoir effectuer un mouvement de translation dans un plan formant un angle (δ) inférieur à 90° avec une direction perpendiculaire à une direction radiale.

11. - Bande de roulement (12) en mélange de caoutchouc, cette bande (12) ayant une surface de roulement (15) limitée axialement par des faces latérales (17) et comportant au moins une cavité (18) dont au moins une partie est située sous la surface de roulement (15) et qui débouche sur au moins une face latérale (17) de cette même bande de roulement (12), cette bande (12) étant pourvue d'une surface de jonction (32 ; 33 ; 34 ; 35 ; 36) s'étendant entre la surface d'au moins une cavité (18) et la surface de roulement (15), cette bande (12) étant **caractérisée en ce que** la longueur de la trace de la surface de jonction (32 ; 33 ; 34 ; 35 ; 36), dans un plan de coupe perpendiculaire à la surface de roulement (15) et à la direction axiale, mesurée entre la surface de la cavité (18) et la surface de roulement (15) est supérieure à la distance minimale (22 ; 23 ; 24 ; 25 ; 26) entre la surface de la cavité (18) et ladite surface de roulement (15).

12. - Bande de roulement (12) selon la revendication 11, **caractérisée en ce que** la différence entre la longueur de ladite trace et la distance minimale (22 ; 23 ; 24 ; 25 ; 26) entre la surface de la cavité (18) et la surface de roulement (15) est supérieure à 20 % de ladite distance minimale (22 ; 23 ; 24 ; 25 ; 26).

## Claims

1. Mould (1) for moulding a tread (12) made from a rubber mix, this tread (12) having a running surface (15) bounded axially by lateral faces (17), the mould (1) comprising at least one part (16) for moulding the running surface (15), at least two lateral parts (19) for moulding the lateral faces (17) and at least one pin (5; 51; 52; 53; 54; 55; 56) for moulding a cavity (18) inside the tread (12), this pin, when viewed in a section plane perpendicular to the mould face (16) that moulds the running surface (15) and to the axial direction, having a surface whose contour is formed by a first contour portion (151; 152; 153; 154) and a second contour portion (251; 252; 253; 254), these contour portions extending between the contour point (71; 72; 73; 74) located closest to, and the contour point (61; 62; 63; 64) located furthest away from the mould face (16) that moulds the running surface (15), the said pin (5; 51; 52; 53; 54; 55; 56) being positioned at least partially below the face (16) that moulds the running surface (15) and being connected to at least one mould face that moulds at least part of a lateral face (17) of the tread (12), such that during moulding the pin (5; 51; 52; 53; 54; 55; 56) produces two flows of the rubber mix which envelop the pin (5; 51; 52; 53; 54; 55; 56) and reunite to form a junction surface (32; 33; 34; 35; 36), the said mould being **characterised in that** at least one pin (5; 51; 52; 53; 54; 55; 56) has means to render the mix flows asymmetrical so that the junction surface (32; 33; 34; 35; 36), viewed in the said plane, is such that the length of the trace of the junction surface (32; 33; 34; 35; 36) on the said plane, measured between the surface of the pin (5; 51; 52; 53; 54; 55; 56) and the mould face (16) that moulds the running surface (15), is greater than the minimum distance (22; 23; 24; 25; 26) between the surface of the pin (5; 51; 52; 53; 54; 55; 56) and the said face (16) that moulds the running surface (15).

2. Mould according to Claim 1, **characterised in that** the length of the said first portion (151; 152; 153; 154) is different from the length of the said second portion (251; 252; 253; 254).

3. Mould according to Claim 2, **characterised in that** the difference between the length of the first contour portion (151; 152; 153; 154) and the length of the second contour portion (251; 252; 253; 254) is at least equal to 10% of the length of the shorter contour portion.

4. Mould according to any of Claims 1 to 3, **characterised in that** one of the contour portions is provided with a mean surface roughness greater than the mean surface roughness of the other contour portion.

5. Mould according to Claim 4, **characterised in that** the difference between the mean surface roughnesses Ra of the first contour portion (151; 152; 153; 154) and the second contour portion (251; 252; 253; 254) is at least equal to 2 microns.

6. Mould according to any of Claims 1 to 5, **characterised in that** at least one contour portion is at least partly covered by a coating so as to produce a difference in the slip velocity of the mix on the said contour portions.

7. Mould according to any of Claims 1 to 6, **characterised in that** means are also provided for producing a mean temperature difference between the surface of the first contour portion (151; 152; 153; 154) and the surface of the second contour portion (251; 252; 253; 254) of at least one pin (5; 51; 52; 53; 54; 55; 56).

8. Mould according to Claim 7, **characterised in that** the said temperature difference is equal to at least 20°C.

9. Mould according to Claim 1, **characterised in that** at least one pin (5; 51; 52; 53; 54; 55; 56) for moulding a cavity (18) has additional means for causing it to rotate essentially about its longitudinal axis.

10. Mould according to any of Claims 1 to 9, **characterised in that** at least one pin (5; 51; 52; 53; 54; 55; 56) is mounted so that it can move axially and radially relative to the mould, such that it can undergo a translation movement in a plane forming an angle (δ) smaller than 90° relative to a direction perpendicular to a radial direction.

11. Tread (12) made from a rubber mix, this tread (12) having a running surface (15) bounded axially by lateral faces (17) and comprising at least one cavity (18) at least part of which is located below the running surface (15) and which opens onto at least one lateral face (17) of the said tread (12), the tread (12) being provided with a junction surface (32; 33; 34; 35; 36) that extends between the surface of at least one cavity (18) and the running surface (15), the said tread (12) being **characterised in that** the length of the trace of the junction surface (32; 33; 34; 35; 36) in a section plane perpendicular to the running surface (15) and to the axial direction, measured between the surface of the cavity (18) and the running surface (15), is greater than the minimum distance (22; 23; 24; 25; 26) between the surface of the cavity (18) and the said running surface (15).

12. Tread (12) according to Claim 11, **characterised in that** the difference between the length of the said trace and the minimum distance (22; 23; 24; 25; 26) between the surface of the cavity (18) and the running surface (15) is larger than 20% of the said minimum distance (22; 23; 24; 25; 26).

## Patentansprüche

1. Form (1) zum Formen eines Laufstreifens (12) aus einer Kautschukmischung, wobei dieser Laufstreifen (12) eine axial durch Seitenflächen (17) begrenzte Lauffläche (15) besitzt und diese Form (1) zumindest einen Teil (16) zum Formen der Lauffläche (15), zumindest zwei Seitenteile (19) zum Formen der Seitenflächen (17) und zumindest einen Finger (5; 51; 52; 53; 54; 55; 56) zum Formen eines Hohlraums (18) im Innern des Laufstreifens (12) aufweist, wobei dieser Finger, betrachtet in einer Schnittebene, die senkrecht zu der die Lauffläche formenden Oberfläche (16) und der axialen Richtung steht, eine Oberfläche besitzt, deren Kontur sich aus einem ersten (151; 152; 153; 154) und einem zweiten Konturteil (251; 252; 253; 254) zusammensetzt, und wobei sich diese Konturteile zwischen dem Konturpunkt, der der Oberfläche (16), die die Lauffläche (15) formt, am nächsten (71; 72; 73; 74) ist, und dem Konturpunkt, der am weitesten von ihr entfernt (61; 62; 63; 64) ist erstrecken, wobei sich dieser Finger (5; 51; 52; 53; 54; 55; 56) zumindest teilweise unter der die Lauffläche (15) formenden Oberfläche (16) befindet und mit zumindest einer, zumindest einen Teil einer Seitenfläche (17) des Laufstreifens (12) formenden Fläche verbunden ist, wobei der Finger (5; 51; 52; 53; 54; 55; 56) während des Formens zwei Ströme der Kautschukmischung erzeugt, die den Finger (5; 51; 52; 53; 54; 55; 56) einhüllen und sich unter Bildung einer Nahtfläche (32; 33; 34; 35; 36) wieder vereinen, wobei die Form **dadurch gekennzeichnet ist, daß** mindestens ein Finger (5; 51; 52; 53; 54; 55; 56) Mittel aufweist, um die Ströme der Mischung asymmetrisch zu machen, so daß die Nahtfläche (32; 33; 34; 35; 36) in der genannten Ebene betrachtet so ist, daß die Länge des Verlaufs dieser Nahtfläche (32; 33; 34; 35; 36) in der genannten Ebene, gemessen zwischen der Oberfläche des Fingers (5; 51; 52; 53; 54; 55; 56) und der die Lauffläche (15) formenden Oberfläche (16) größer ist als der minimale Abstand (22; 23; 24; 25; 26) zwischen der Oberfläche des Fingers (5; 51; 52; 53; 54; 55; 56) und der die Lauffläche (15) formenden Oberfläche (16).

2. Form nach Anspruch 1, **dadurch** charakterisiert, daß sich die Länge des ersten Konturteils (151; 152; 153; 154) von der Länge des zweiten Konturteils (251; 252; 253; 254) unterscheidet.

3. Form nach Anspruch 2, **dadurch** charakterisiert, daß der Unterschied zwischen der Länge des ersten Konturteils (151; 152; 153; 154) und der Länge des zweiten Konturteils (251; 252; 253; 254) zumindest 10 % der Länge des kürzeren Konturteils beträgt.

4. Form nach einem der Ansprüche 1 bis 3, **dadurch** charakterisiert, daß einer der Konturteile mit einer mittleren Rauhigkeit versehen ist, die größer ist als die mittlere Rauhigkeit des anderen Konturteils.

5. Form nach Anspruch 4, **dadurch** charakterisiert, daß der Unterschied zwischen den mittleren Rauhigkeiten Ra des ersten Konturteils (151; 152; 153; 154) und des zweiten Konturteils (251; 252; 253; 254) zumindest 2 µm beträgt.

6. Form nach einem der Ansprüche 1 bis 5, **dadurch** charakterisiert, daß zumindest ein Konturteil zumindest teilweise von einem Überzug bedeckt ist, um einen Unterschied der Gleitgeschwindigkeit der Mischung auf den genannten Konturteilen zu erhalten.

7. Form nach einem der Ansprüche 1 bis 6, **dadurch** charakterisiert, daß darüber hinaus Mittel vorgesehen sind, um zwischen der Oberfläche des ersten Konturteils (151; 152; 153; 154) und der Oberfläche des zweiten Konturteils (251; 252; 253; 254) zumindest eines Fingers (5; 51; 52; 53; 54; 55; 56) einen Unterschied der mittleren Temperatur zu erzeugen.

8. Form nach Anspruch 7, **dadurch** charakterisiert, daß der Temperaturunterschied mindestens 20 °C beträgt.

9. Form nach Anspruch 1, **dadurch** charakterisiert, daß zumindest ein Finger (5; 51; 52; 53; 54; 55; 56) zum Formen eines Hohlraums (18) über zusätzliche Mittel verfügt, um ihn in eine Drehbewegung in etwa um seine longitudinale Achse zu versetzen.

10. Form nach einem der Ansprüche 1 bis 9, **dadurch** charakterisiert, daß zumindest ein Finger (5; 51; 52; 53; 54; 55; 56) relativ zur Form axial und radial beweglich eingebaut ist, um eine Translationsbewegung ausführen zu können, die in einer Ebene liegt, die einen Winkel (δ) kleiner als 90° zu einer senkrecht zu einer radialen Richtung stehenden Richtung einschließt.

11. Laufstreifen (12) aus einer Kautschukmischung, wobei dieser Laufstreifen (12) eine axial durch Seitenflächen (17) begrenzte Lauffläche (15) besitzt und zumindest einen Hohlraum (18) aufweist, der sich zumindest teilweise unter der Lauffläche (15) befindet und der zumindest zu einer Seitenfläche (17) des selben Laufstreifens (12) hin offen ist, wobei dieser Laufstreifen (12) mit einer Nahtfläche (32; 33; 34; 35; 36) versehen ist, die sich zwischen der Oberfläche zumindest eines Hohlraums (18) und der Lauffläche (15) erstreckt, wobei dieser Laufstreifen (12) **dadurch** charakterisiert ist, daß die Länge des Verlaufs der Nahtfläche (32; 33; 34; 35; 36) in einer senkrecht zur Lauffläche (15) und zur axialen Richtung stehenden Schnittebene, gemessen zwischen der Oberfläche des Hohlraums (18) und der Lauffläche (15) größer ist als der minimale Abstand (22; 23; 24; 25; 26) zwischen der Oberfläche des Hohlraums (18) und der Lauffläche (15).

12. Laufstreifen (12) nach Anspruch 11, **dadurch** charakterisiert, daß der Unterschied zwischen der Länge des genannten Verlaufs und dem minimalen Abstand (22; 23; 24; 25; 26) zwischen der Oberfläche des Hohlraums (18) und der Lauffläche (15) größer als 20 % des genannten minimalen Abstands (22; 23; 24; 25; 26) ist.
